(19) Europäisches Patentamt
European Patent Office
Office européen des brevets

(11) **EP 1 913 867 A1**

(12) **EUROPEAN PATENT APPLICATION**

(43) Date of publication:
**23.04.2008 Bulletin 2008/17**

(51) Int Cl.:
*A61B 5/00* (2006.01)   *G02B 6/036* (2006.01)

(21) Application number: **07020421.9**

(22) Date of filing: **18.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **19.10.2006 JP 2006284449**

(71) Applicant: **Sumitomo Electric Industries, Ltd.**
**Osaka-shi, Osaka 541-0041 (JP)**

(72) Inventor: **Nakaji, Haruo**
**Yokohama-shi**
**Kanagawa 244-8588 (JP)**

(74) Representative: **HOFFMANN EITLE**
**Patent- und Rechtsanwälte**
**Arabellastrasse 4**
**81925 München (DE)**

(54) **Optical measuring device and optical measuring method**

(57)    An object of the present invention is to provide an optical measuring device and optical measuring method capable of locally irradiating a measuring object with light and efficiently receiving reflection, scattered light, fluorescence, etc. from the measuring object.

An optical measuring device according to the present invention has a light emitting portion; an irradiation waveguide for guiding light from the light emitting portion toward an object; a receiving waveguide for guiding light from the object; and a light receiving portion for receiving the light having been guided through the receiving waveguide; and is characterized in that a numerical aperture at a receiving end of the receiving waveguide is larger than a numerical aperture at an emission end of the irradiation waveguide.

**Fig.1**

## Description

### BACKGROUND OF THE INVENTION

#### Field of the Invention

The present invention relates to a device irradiating a measuring object with light and measuring reflection, scattered light, fluorescence, etc. from the measuring object, thereby measuring a property of the measuring object or the like, and a measuring method thereof.

#### Related Background Art

A known optical measuring device of this type is, for example, the one described in Patent Document 1. This document describes a biomedical tissue oxygen monitor using near-infrared spectroscopy and discloses the device having an optical fiber for guiding light to a measuring object to irradiate it with the light, and an optical fiber for receiving reflection, scattered light, fluorescence, etc. from the measuring object and guiding the received light to a light receiving element.

[Patent Document 1] Japanese Patent Application Laid-open No. 5-212016

[Disclosure of the Invention]

[Problem to be Solved by the Invention]

In the optical measuring device as described above, however, the same optical fibers or the optical fibers of the same kind were used in spite of the difference between the characteristics required for the optical fiber for guiding the light to the measuring object and the characteristics required for the optical fiber for receiving the reflection, scattered light, and fluorescence from the measuring object, and it was not successful in adequately achieving local irradiation on the measuring object with the light and efficient reception of the reflection, scattered light, fluorescence, etc. from the measuring object. The present invention has been accomplished in order to solve the above problem.

### SUMMARY OF THE INVENTION

[Means for Solving the Problem]

An optical measuring device according to the present invention is an optical measuring device comprising: a light emitting portion; an irradiation waveguide for guiding light from the light emitting portion and emitting the light toward an object; a receiving waveguide for receiving light from the object and guiding the light; and a light receiving portion for receiving the light having been guided through the receiving waveguide; wherein a numerical aperture at a receiving end of the receiving waveguide is larger than a numerical aperture at an emission end of the irradiation waveguide.

In this optical measuring device, since the light can be emitted toward the measuring object from the irradiation waveguide with the smaller numerical aperture at the emission end of light, the measuring object can be locally irradiated with the light; and since the reflection, scattered light, fluorescence, etc. from the measuring object can be received by the receiving waveguide with the larger numerical aperture at the receiving end, these beams of light can be efficiently received.

The optical measuring device according to the present invention is further characterized in that the receiving end of the receiving waveguide is located near the emission end of the irradiation waveguide.

In this optical measuring device, since the receiving end of the receiving waveguide is located near the emission end of the irradiation waveguide, the receiving waveguide is able adequately to receive the light from the measuring object which tends to easily spread over the surrounding area.

The optical measuring device according to the present invention is further characterized in that a portion near the emission end of the irradiation waveguide and a portion near the receiving end of the receiving waveguide are integrated.

In this optical measuring device, since the portion near the emission end of the irradiation waveguide and the portion near the receiving end of the receiving waveguide are integrated, it becomes easier to locate the receiving end near the measuring object to be irradiated with the light whereby the receiving waveguide can adequately receive the light appearing from the measuring object.

The optical measuring device according to the present invention is further characterized in that the irradiation waveguide and the receiving waveguide are made of their respective materials with different refractive indices and are formed in such an integral structure that axes thereof are substantially parallel to each other.

In this optical measuring device, the waveguides can be constructed in a more compact form and handling thereof is easy during measurement.

The optical measuring device according to the present invention is further characterized in that the irradiation waveguide and the receiving waveguide are formed in a coaxial layer structure.

In this optical measuring device, it is easier to implement the integration of the waveguides and the device can be constructed in a more compact configuration.

An optical measuring method according to the present invention is an optical measuring method comprising guiding light from a light emitting portion and emitting the light toward an object by a first waveguide with a small numerical aperture at an end, and receiving light from the object and guiding the light to a light receiving portion by a second waveguide with a large numerical aperture at an end.

In this optical measuring method, since the first waveguide with the small numerical aperture is used to guide the light toward the object to irradiate the object, the object can be locally irradiated; and since the second waveguide with the large numerical aperture is used to receive the light form the measuring object, the light can be efficiently received.

[Effect of the Invention]

The present invention enables the local irradiation on the measuring object with the light and the efficient reception of the light from the measuring object, and the present invention is thus successful in achieving relative improvement in reception amount and improvement in measurement accuracy.

BRIEF DESCRIPTION OF THE DRAWINGS

Fig. 1 is a schematic diagram of an optical measuring device 1 of the first embodiment and a measuring method using the same.
Fig. 2 is a drawing showing a sectional structure of irradiation and receiving waveguides in the optical measuring device of the first embodiment.
Fig. 3 is a drawing showing a sectional structure near one end faces of irradiation and receiving waveguides in an optical measuring device of the second embodiment
Fig. 4 is a schematic diagram showing a concept of an optical measuring method using the optical measuring device of the second embodiment.
Fig. 5 is a drawing showing a sectional structure of irradiation and receiving waveguides in an optical measuring device of the third embodiment.
Fig. 6 is a schematic diagram showing a concept of an optical measuring method using the optical measuring device of the third embodiment.

[Description of Reference Symbols]

1    optical measuring device
2    light emitting portion
3    light emitting element
4    emission controller
5    light receiving portion
6    light receiving element
7    reception controller
8    irradiation waveguide
9    receiving waveguide
10   measuring object
11   first core part
12   second core part
13   cladding part
14   protecting layer

DESCRIPTION OF THE PREFERRED EMBODIMENTS

The best mode for carrying out the present invention will be described below in detail with reference to the accompanying drawings. The same elements will be denoted by the same reference symbols throughout the description of the drawings, without redundant description.

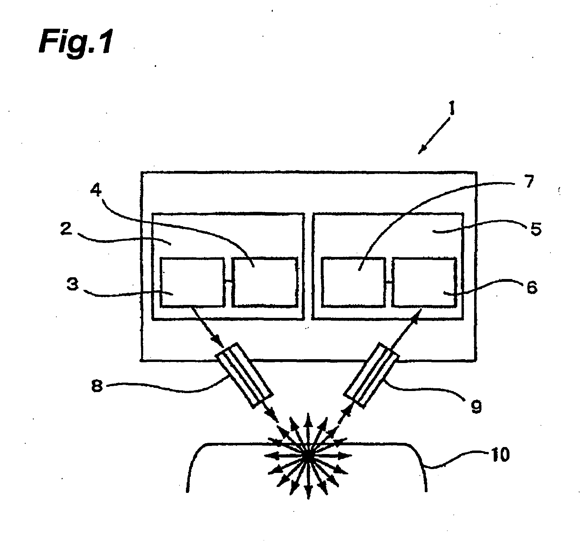

Fig. 1 is a schematic diagram of an optical measuring device 1 of the first embodiment and a measuring method using it The optical measuring device 1 has a light emitting portion 2, an irradiation waveguide 8 for guiding light from the light emitting portion toward a measuring object, a receiving waveguide 9 for receiving reflection, scattered light, fluorescence, etc. from the measuring object and guiding the received light, and a light receiving portion 5 for receiving the light having been guided through the receiving waveguide 9.

The light emitting portion 2 has a light emitting element 3 and a controller 4 for it, and light emitted from the light emitting element 3 is optically coupled to the irradiation waveguide 8 and guided in the irradiation waveguide 8. The light emitting element 3 is an element for outputting light and preferably includes a semiconductor laser element for outputting a laser beam.

The light receiving portion 5 has a light receiving element 6 and a controller 7 for it, and light having been guided through the receiving waveguide 9 is optically coupled to the light receiving element 6. The light receiving element 6 to be used is a photodiode or the like.

An emission end of the irradiation waveguide 8 and a receiving end of the receiving waveguide 9 are disposed in proximity on the same side so that each of them faces a measuring object 10, and the numerical aperture at the receiving end of the receiving waveguide 9 is set to be larger than the numerical aperture at the emission end of the irradiation waveguide 8. This configuration permits the light emitted toward the measuring object to be locally projected, and permits the receiving waveguide to efficiently receive light generated at various angles from the measuring object.

The light emerging from the irradiation waveguide 8 is radiated onto the measuring object 10 and the measuring object 10 emits reflection, scattered light, fluorescence, etc. according to the wavelength, intensity, etc. of the irradiating light. Since these beams of light emitted from the measuring object are normally omnidirectional, they are emitted at various angles in all directions. On the other hand, the light received and guided by the receiving waveguide 9 is limited to light incident at angles equal to or smaller than the critical angle of incidence determined by the structure of the receiving waveguide 9, to the end face of the receiving waveguide 9. The critical angle of incidence $\theta_{imax}$ is represented by the formula below, where $n_j$ is the refractive index of the portion where the light is guided through the receiving waveguide 9 and $n_0$ is the refractive index of the sur-

roundings.
[Mathematical Formula 1]

$$\sin(\theta_{imax}) = \sqrt{n_j^2 - n_0^2}$$

On the other hand, the numerical aperture of the receiving waveguide 9 ($NA_0$) is represented by the following formula.
[Mathematical Formula 2]

$$NA_0 = \sqrt{n_j^2 - n_0^2}$$

From the above two formulae, the following approximation holds between the critical angle of incidence $\theta_{imax}$ and the numerical aperture ($NA_0$).
$$\theta_{imax} = NA_0$$

Namely, since the critical angle of incidence $\theta_{imax}$ in the receiving waveguide 9 increases with increase in $NA_0$ of the receiving waveguide, the receiving waveguide 9 becomes able to receive light incident to the end face thereof at wider angles and to guide the received light to the light receiving element 6. This enables the measuring device to receive a greater amount of the light emitted from the measuring object 10 and thus to obtain a higher volume of information.

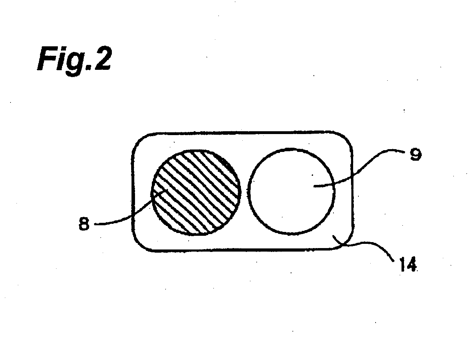

Fig. 2 is a drawing showing a sectional configuration near one end faces of the irradiation waveguide 8 and the receiving waveguide 9 in the optical measuring device 1 of the first embodiment. The irradiation waveguide 8 and the receiving waveguide 9 both are comprised of optical fibers whose cross section is almost circular, the irradiation waveguide 8 and the receiving waveguide 9 are located in proximity to each other, and these optical fibers are bundled to be integrated by a protecting layer 14 so that the sectional shape is nearly rectangular in the size of 150 $\mu$m $\times$ 250 $\mu$m. The irradiation waveguide 8 is an optical fiber made of pure silica glass and in a nearly circular cylinder shape with the diameter of 60 $\mu$m, and the numerical aperture at the end thereof is 0.10. The receiving waveguide 9 is an optical fiber made of germanium-doped silica glass and in a nearly circular cylinder shape with the diameter of 60 $\mu$m, and the numerical aperture at the end thereof is 0.20.

The description will continue again based on Fig. 1. The light emitting portion 2 includes a semiconductor laser element capable of emitting light with the center wavelength of 700 nm, and is optically coupled to the irradiation optical fiber on the other end side opposite to the integrated end. On the other hand, the light receiving portion includes a photodiode capable of receiving light with the center wavelength of 700 nm, and is optically coupled to the receiving optical fiber on the other end side opposite to the integrated end. In this configuration, the light with the center wavelength of 700 nm emitted from the light emitting portion 2 is guided through the irradiation optical fiber and emitted from the integrated end side toward the measuring object. The light emitted from the measuring object is received by the receiving optical fiber and is guided to the photodiode in the light receiving portion. Furthermore, the received light is converted into an electric signal by the photodiode. In a preferred configuration, a lens (not shown) or the like is interposed according to need to improve the coupling efficiency in the optical coupling between these semiconductor laser element and irradiation optical fiber or in the optical coupling between the photodiode and the receiving optical fiber.
In the present embodiment, the irradiation end of the irradiation waveguide 8 and the receiving end of the receiving waveguide 9 are disposed in proximity and integrated, whereby the irradiation end and the receiving end can be disposed virtually at the same position; as a result, the device is able to efficiently receive the light emitted from near the irradiated part in the measuring object and handling thereof also becomes easier. Furthermore, the present embodiment is suitably applicable to cases where the measuring object itself is always moving like a living body, and cases where it is necessary to locally radiate light and to receive light through a limited opening range, for example, in vivo cases.

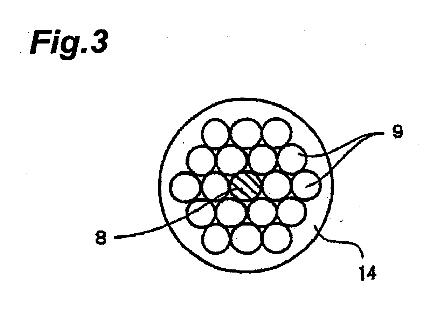

Fig. 3 is a drawing showing a sectional structure near one end faces of an irradiation waveguide and receiving waveguides in an optical measuring device of the second embodiment. The irradiation waveguide 8 and the receiving waveguides 9 both are optical fibers whose cross section is nearly circular, the plurality of receiving waveguides are arranged to surround the periphery of the irradiation waveguide so that their axes are nearly parallel to each other, and these optical fibers are integrated. The irradiation optical fiber is made of pure silica glass and in a nearly circular cylinder shape with the diameter of 60 $\mu$m, and the numerical aperture at the end thereof is 0.10. The receiving optical fibers are made of germanium-doped silica glass and in a nearly circular cylinder shape with the diameter of 60 $\mu$m, and the numerical aperture at the end thereof is 0.2. Namely, the irradiation and receiving optical fibers are made of the respective materials with different refractive indices, the numerical aperture of the irradiation optical fiber is smaller, and the receiving optical fibers with the larger numerical aperture are arranged around the irradiation optical fiber. In the example of Fig. 3, totally seventeen receiving optical fibers are located in immediate proximity to the periphery of one irradiation optical fiber, and these are covered by the protecting layer 14 to be integrated so that the sectional shape thereof becomes nearly circular in the diameter of about 400 μm as a whole.

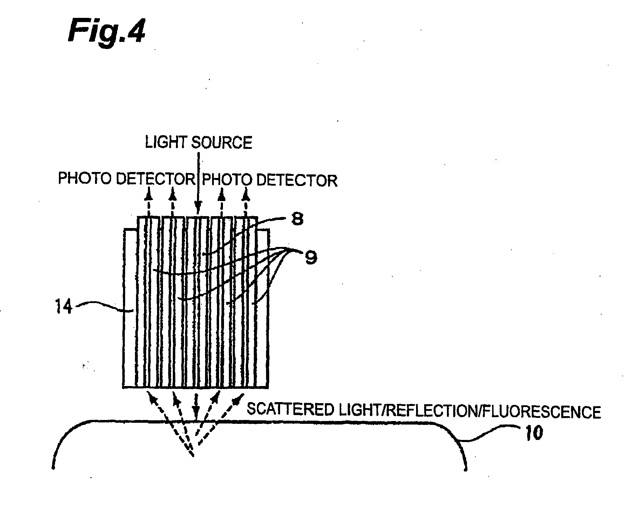

Fig. 4 is a schematic diagram showing a concept of an optical measuring method using the optical measuring device according to the second embodiment. As with the first embodiment, the light emitting portion includes a semiconductor laser element capable of emitting light with the center wavelength of 700 nm and is optically coupled to the irradiation optical fiber on the other end side opposite to the integrated end. On the other hand, the light receiving portion includes totally seventeen photodiodes capable of receiving light with the center wavelength of 700 nm, and they are optically coupled to the respective optical fibers on the other end side opposite to the integrated ends of the receiving optical fibers. The irradiation optical fiber 8 and the receiving optical fibers 9 are not integrated on the other end side opposite to the integrated ends, and are optically connected to the light emitting portion or to the light receiving portion, respectively. In a preferred configuration, a lens (not shown) or the like is interposed according to need to improve the coupling efficiency in the optical coupling between these semiconductor laser element and irradiation optical fiber or in the optical coupling between the photodiodes and the receiving optical fibers.

The light emitted from the irradiation optical fiber 8 with the smaller numerical aperture located in the central region is radiated onto the measuring object 10. The light emerging from the end face of the irradiation optical fiber 8 is emitted with a divergence angle determined by the maximum (incidence) critical angle of the irradiation optical fiber, but the divergence angle is small because of the small numerical aperture; therefore, a specific part of the measuring object 10 can be locally irradiated with the light.

On the other hand, since the emission (reflection, scattered light, fluorescence, etc.) from the measuring object 10 based on the light radiated onto the measuring object 10 is received by the receiving optical fibers 9 with the larger numerical aperture located around the irradiation optical fiber, the receiving optical fibers are able to receive the light emitted at various angles from the measuring object 10. Furthermore, since the end faces being the light receiving surfaces of the receiving fibers are located around the emission end of the irradiation optical fiber, the receiving fibers are also able to receive the light in a wider range in terms of space. In addition, when the irradiation and receiving optical fibers are arranged as described above, it is feasible to make smaller the cross section of the part not contributing to irradiation and reception, to achieve miniaturization of the device, and to facilitate handling. Furthermore, the irradiation end and the receiving ends can be located virtually at the same position, and the present embodiment is suitably applicable to cases where the measuring object itself is always moving like a living body and cases where it is necessary to locally radiate light and to receive light through a limited opening range, e.g., in vivo cases.

The light with the center wavelength of 700 nm emitted from the light emitting portion is guided through the irradiation optical fiber 8 and is radiated from the bundled end side toward the measuring object 10. Furthermore, the light emitted from the measuring object 10 is received and guided by the receiving optical fibers 9 and optically coupled to the photodiodes in the light receiving portion to be converted into an electric signal. A property of the measuring object can be measured by analyzing the electric signal to detect a change in intensity of light at a specific wavelength or to perform a spectral analysis. Examples of such measurement include measurement of the hemoglobin content of blood based on measurement of intensity of transmitted light by blood, measurement of a fluorescence characteristic from a cancer tissue with a fluorescent substance aggregated, and so on.

In the above-described example, the totally seventeen receiving optical fibers are located in immediate proximity to the periphery of one irradiation optical fiber, but the present invention is not limited to this form; it is a matter of course that a plurality of receiving optical fibers may be located around a plurality of irradiation optical fibers.

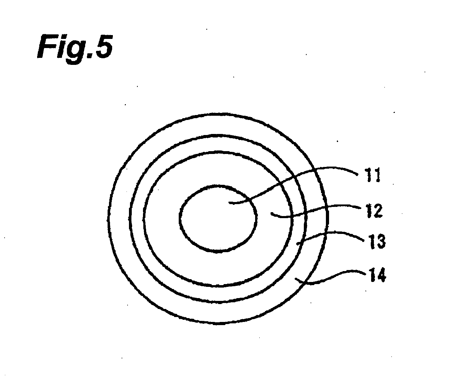

Fig. 5 is a drawing showing a sectional structure of irradiation and receiving waveguides in an optical measuring device of the third embodiment. The present embodiment uses an optical fiber in which a plurality of layers with a cross section of a nearly circular shape are integrated; the optical fiber is made in a coaxial three-layered structure having a first core near the central region, a second core around it, and a cladding around the second core, and each layer is made of a material whose principal ingredient is silica glass. The first core part has the diameter of 6 μm, the second core part the diameter of 125 μm, and the cladding part the diameter of 250 μm, and the periphery of the cladding part is covered by a resin layer about 75 μm thick. The first core part is made of germanium-doped silica glass, the second core part of pure silica glass, and the cladding part of fluorine-doped silica glass.

The numerical aperture for light guided in the first core part is 0.05, and the numerical aperture for light guided in the second core part is 0.07; therefore, the numerical aperture for the light guided in the second core part is larger than the numerical aperture for the light guided in the first core part.

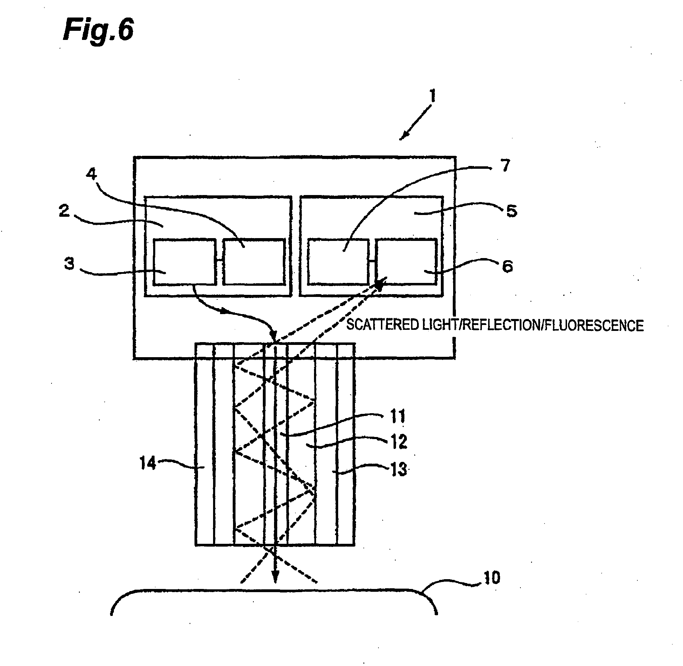

Fig. 6 is a schematic diagram showing the concept of the optical measuring device and the optical measuring method using it according to the third embodiment. The light receiving portion and the light emitting portion have the respective configurations similar to those in the first and second embodiments, and, using the waveguides in the structure shown in Fig. 5, the light emitted from the light emitting portion is optically coupled to the first core part 11 and guided to be emitted toward the measuring object 10. The light emitted from the measuring object 10 is received by the second core part 12, and guided to be optically coupled to the light receiving element 6. Since the numerical aperture for the light guided in the first core part 11 is smaller, a local part of the measuring object 10 can be irradiated with the light emitted, guided in the first core part 11, and radiated onto the measuring object 10. Since the numerical aperture for the light guided in the second core part 12 is larger, the second core part is able to collect the light emitted at various angles from near the irradiated part of the measuring object 10 and as a result, the device is able to collect a higher volume of information from the measuring object 10.

When the waveguides are formed in the structure as shown in Fig. 5, it becomes easier to integrate the irradiation and receiving waveguides. Since the irradiation end and receiving end can be readily located virtually at the same position, the present embodiment is suitably applicable to cases where the measuring object itself is always moving like a living body and cases where it is necessary to locally radiate light and to receive light through a limited opening range, e.g., in vivo cases.

The materials for making up the first core part 11 and the second core part 12 may be properly selected from appropriate materials in consideration of easiness of production and others so that the numerical aperture for the light guided in the second core part becomes larger than the numerical aperture for the light guided in the first core part. Furthermore, the cross section of the end face part of the receiving waveguide is preferably larger than that of the irradiation waveguide, in order to enable reception of light from a spatially wide range; i.e., the cross section of the end face of the second core part is preferably larger than that of the first core part.

It is noted that the present invention is by no means limited to the above embodiments. For example, the waveguides in each of the above embodiments were the optical fibers of glass, but the waveguides can be made of resin; generally, the waveguides may be made of any material that is highly transparent to the irradiating light and the received light.

[Industrial Applicability]

As described above, the present invention is applicable to apparatus for irradiating a measuring object with light and measuring reflection, scattered light, fluorescence, etc. from the measuring object, thereby measuring a property of the measuring object or the like, and measuring methods thereof.

**Claims**

1. An optical measuring device comprising:

a light emitting portion;
an irradiation waveguide for guiding light from the light emitting portion and emitting the light toward an object;
a receiving waveguide for receiving light from the object and guiding the light; and
a light receiving portion for receiving the light having been guided through the receiving waveguide;

wherein a numerical aperture at a receiving end of the receiving waveguide is larger than a numerical aperture at an emission end of the irradiation waveguide.

2. The optical measuring device according to claim 1, wherein the receiving end of the receiving waveguide is located near the emission end of the irradiation waveguide.

3. The optical measuring device according to claim 2, wherein a portion near the emission end of the irradiation waveguide and a portion near the receiving end of the receiving waveguide are integrated.

4. The optical measuring device according to claim 2 or 3, wherein the irradiation waveguide and the receiving waveguide are made of their respective materials with different refractive indices and are formed in such an integral structure that axes thereof are substantially parallel to each other.

5. The optical measuring device according to claim 4, wherein the irradiation waveguide and the receiving waveguide are formed in a coaxial layer structure.

6. An optical measuring method comprising guiding light from a light emitting portion and emitting the light toward an object by a first waveguide with a small numerical aperture at an end, and receiving light from the object and guiding the light to a light receiving portion by a second waveguide with a large numerical aperture at an end.

*Fig.1*

EP 1 913 867 A1

*Fig.2*

8

9

14

# Fig.3

## Fig.4

LIGHT SOURCE

PHOTO DETECTOR | PHOTO DETECTOR

8

9

14

SCATTERED LIGHT/REFLECTION/FLUORESCENCE

10

**Fig.5**

## Fig.6

SCATTERED LIGHT/REFLECTION/FLUORESCENCE

**European Patent Office**

**EUROPEAN SEARCH REPORT**

Application Number

EP 07 02 0421

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/01745 A (LJ LAB L L C [US]; JUNG WAYNE D [US]; JUNG RUSSELL W [US]; LOUDERMILK) 14 January 1999 (1999-01-14) * page 43, line 14 - page 46, line 15 * | 1-3,6 | INV. A61B5/00 G02B6/036 |
| X | JP 63 302305 A (HITACHI LTD; HITACHI AUTOMOTIVE ENG) 9 December 1988 (1988-12-09) | 1-4,6 | |
| Y | *PAJ Abstract* | 5 | |
| X | US 2006/158655 A1 (EVERETT MATTHEW J [US] ET AL) 20 July 2006 (2006-07-20) | 1-3,6 | |
| Y | * paragraphs [0029] - [0040]; figure 1 * | 5 | |

TECHNICAL FIELDS SEARCHED (IPC)

A61B
G02B

The present search report has been drawn up for all claims

| Place of search | Date of completion of the search | Examiner |
|---|---|---|
| Munich | 5 March 2008 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 02 0421

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-2008

| Patent document cited in search report | | Publication date | Patent family member(s) | | Publication date |
|---|---|---|---|---|---|
| WO 9901745 | A | 14-01-1999 | AU | 757807 B2 | 06-03-2003 |
| | | | AU | 8380298 A | 25-01-1999 |
| | | | BR | 9810655 A | 30-10-2001 |
| | | | CA | 2294880 A1 | 14-01-1999 |
| | | | EP | 0993601 A1 | 19-04-2000 |
| | | | JP | 2002508076 T | 12-03-2002 |
| | | | US | 5926262 A | 20-07-1999 |
| JP 63302305 | A | 09-12-1988 | NONE | | |
| US 2006158655 | A1 | 20-07-2006 | EP | 1838212 A1 | 03-10-2007 |
| | | | WO | 2006077045 A1 | 27-07-2006 |

EPO FORM P0459

For more details about this annex : see Official Journal of the European Patent Office, No. 12/82

**EP 1 913 867 A1**

## REFERENCES CITED IN THE DESCRIPTION

**Patent documents cited in the description**

- JP 5212016 A